# EUROPEAN PATENT SPECIFICATION

(11) **EP 4 448 085 B1**
(45) Date of publication and mention of the grant of the patent: **01.10.2025**
(21) Application number: 22830508.2
(22) Date of filing: 06.12.2022
(51) Int. Cl.: A61N 1/375, A61N 1/368, A61N 1/365

(54) **SYSTEM COMPRISING AT LEAST TWO DEVICES**
SYSTEM MIT MINDESTENS ZWEI VORRICHTUNGEN
SYSTÈME COMPRENANT AU MOINS DEUX DISPOSITIFS

(30) Priority: 15.12.2021 US 202163289784 P; 05.01.2022 EP 22150271
(43) Date of publication of application: 23.10.2024
(73) Proprietor: BIOTRONIK SE & Co. KG, 12359 Berlin (DE)
(72) Inventor: MUESSIG, Dirk, West Linn, Oregon 97068 (US); STOTTS, Larry, Fulshear, Texas 77441 (US); MIDGETT, Madeline Anne, Portland, Oregon 97213 (US); JONES, Christopher, Oregon City, Oregon 97045 (US); WHITTINGTON, R. Hollis, Portland, Oregon 97202 (US)
(74) Representative: Biotronik Corporate Services SE
(86) International application number: PCT/EP2022/084522
(87) International publication number: WO 2023/110516

(56) References cited:
- US-A1- 2018 021 585
- US-A1- 2020 368 538
- US-A1- 2021 236 827
- US-A1- 2021 290 965
- US-B2- 10 881 861

## Description

The invention is generally directed to a system comprising a first intracardiac device for implantation within the heart of a patient and at least one second implantable device, for example an intracardiac device.

Active intracardiac devices, for example implantable intracardiac pacemakers (also known as implantable leadless pacemakers - ILP), are well known miniaturized medical devices which are entirely implanted into a heart chamber, either a ventricle or atrium of a patient. They are considered the future of cardiac pacing. Intracardiac pacemakers are used, for example, for patients who suffer from a bradycardia, with a heart that beats too slow to fulfill the physiological needs of the patient. Intracardiac devices may apply electrical stimulation in the form of pulses to the heart in order to generate a physiologically appropriate heart rate and/or in the form of fast pacing or shocks for cardioversion or defibrillation in order to restore a more normal heart rhythm. Active intracardiac devices may, e.g. deliver antitachycardia pacing (ATP), i.e. pacing the heart with a faster stimulation rate than the tachycardia rate to terminate a tachycardia. Alternative or additional functions of intracardiac devices comprise providing other electrical or electromagnetic signals to the heart or its surrounding tissue and sensing electrical or electromagnetic signals or other physiological parameters of the heart and/or its surrounding tissue. Due to the highly restricted device size, such device has a small battery capacity.

However, there are circumstances in which a patient suffers from various cardiac arrhythmias that require different cardiac therapies. In such cases, a system of implantable devices may be implanted comprising at least two medical devices or units.

Document US 2016/0067490 A1 discloses a dual-chamber, leadless pacing system comprising at least one atrial pacing device and at least one ventricular pacing device. In order to adjust the pacing rate in such system signals are transmitted from the atrial pacing device to the ventricular pacing device or from the ventricular pacing device to the atrial pacing device, wherein the respective pacing rate is adjusted based on the received signal. For that a separate communication unit of the atrial pacing device or the ventricular pacing device that includes suitable hardware (e.g. an antenna), firmware, software or any combination thereof consumes energy while communicating with the respective other pacing device.

As indicated above, the new technology brings a number of challenges with it. Due to the size restrictions of intracardiac devices requirements for energy consumption and use of electronic components have increased drastically. There are solutions available in which a basic VVIR functionality is implemented in a ventricular ILP, in which the rate response functionality is based on an accelerometer. This allows adjusting the ventricular pacing rate in case the patient is in atrial fibrillation. VVI(R) is one of the more commonly used pacing modes. VVI(R) is ventricular demand pacing. The ventricle is paced, sensed, and the pulse generator inhibits pacing output in response to a sensed ventricular event. This mode of pacing prevents ventricular bradycardia and is therefore primarily indicated for patients with atrial fibrillation and a slow ventricular response. However, since the pulse generator only paces and senses in the ventricle, there may be a loss of AV synchrony (i.e. the physiological condition of atrial electrical activity followed by ventricular electrical activity, with the interval between being that necessary for impulse conduction from atria to ventricles), which can potentially lead to pacemaker syndrome.

Intracardiac devices that are intended to be implanted in the heart's atrium require specialized design considerations. The available volume for device placement is smaller compared to the ventricle, and the atrial tissue is significantly thinner compared to the ventricle.

US 2020/368538 A1 discloses implantable systems for performing cardiac resynchronization therapy (CRT), methods for use therewith, and leadless pacemakers for use therewith. Such a system can include a first leadless pacemaker configured to be implanted in or on the right atrial (RA) chamber and selectively pace the RA chamber, a second leadless pacemaker configured to be implanted in or on the right ventricular (RV) chamber and selectively pace the RV chamber, and a third leadless pacemaker configured to be implanted in or on the left ventricular (LV) chamber and selectively pace the LV chamber, wherein one of the leadless pacemaker is designated a master leadless pacemaker. In certain embodiments, the master leadless pacemaker determines a VV delay and an AV delay and coordinates CRT using such delays.

US 2021/236827 A1 discloses systems, devices, and methods for monitoring for atrial capture. Such a method, for use within an implantable system including an atrial leadless pacemaker (aLP) and a ventricular leadless pacemaker (vLP), includes storing within a memory of the vLP a paced atrial activation morphology template corresponding to far-field atrial signal components expected to be present in a vEGM sensed by the vLP when an atrial pacing pulse delivered by the aLP captures atrial tissue. The vLP senses a vEGM and compares a morphology of a portion of the sensed vEGM to the paced atrial activation morphology template to determine whether a match therebetween is detected. Additionally, the vLP determines whether atrial capture occurred or failed to occur (responsive to an atrial pacing pulse), based on whether the vLP detects a match between the morphology of a portion of the sensed vEGM and the paced atrial activation morphology template.

US 2021/290965 A1 an implantable system including a first leadless pacemaker (LP1) implanted in or on a first chamber of a heart and a second leadless pacemaker (LP2) implanted in or on a second chamber of the heart. The LP1 is configured to time delivery of one or more pacing pulses delivered to the first chamber of the heart based on timing of cardiac activity associated with the second chamber of the heart detected by the LP1 itself. The LP1 is also configured to transmit implant-to-implant (i2i) messages to the LP2. The LP2 is configured to time delivery of one or more pacing pulses delivered to the second chamber of the heart based on timing of cardiac activity associated with the second chamber of the heart as determined based on one or more i2i messages received by the LP2 from the LP1.

US 10,881,861 B2 discloses a system that can be used for delivering cardiac resynchronization therapy (CRT). The system may include a pacing device configured to be implanted within a patient. The pacing device can include a plurality of electrodes, signal generation circuitry configured to deliver ventricular pacing via the plurality of electrodes, and a sensor configured to produce a signal that indicates mechanical activity of the heart. Processing circuitry can be configured to identify one or more features of a cardiac contraction within the signal, and determine whether the contraction was a fusion beat based on the one or more features.

US 2018/0021585 A1 discloses a medical system including two or more leadless implantable medical devices, each implanted in a different ventricle of the heart and each including a housing, a first electrode secured relative to the housing, a second electrode secured relative to the housing, and a pressure sensor secured relative to the housing. Each of the leadless implantable medical devices may further include circuitry in the housing operatively coupled to the corresponding first electrode, second electrode, and pressure sensor. The medical system may be configured to determine and store a plurality of pressure-pressure data pairs and/or impedance-impedance data pairs generated by the two or more leadless implantable medical devices, from which a representation of a pressure-pressure loop or volume-volume loop may be determined, to facilitate cardiac resynchronization therapy (CRT), patient health status monitoring, and/or the management of a non-CRT cardiac therapy.

Accordingly, there is the need for implantable intracardiac devices to better cope with the requirements for energy consumption, tissue properties and available space.

The above problem is solved with a system comprising the features of claim 1.

The invention is defined in the appended claims. Aspects, embodiments and examples disclosed herein which do not fall within the scope of the appended claims do not form part of the invention, and are merely provided for illustrative purposes.

In particular, an inventive system comprises a first intracardiac device for implantation within the heart of a patient and at least one second implantable device, for example an intracardiac device. The system may therefore be a dual-chamber system, wherein the first intracardiac device and the at least one second intracardiac device each may form a single chamber device. Alternatively, the first intracardiac device and the at least one second intracardiac device, each may form a dual chamber device. For example, the first intracardiac device may provide a treatment with regard to a ventricle of the patient's heart and may be implanted within the ventricle wall of the heart and the second intracardiac device, for example, may provide a treatment with regard to an atrium of the patient's heart and may be implanted within the atrial wall of the heart. Alternatively, the at least one second device may be another implantable device, for example a device which is located fully within the body or partially within the body and outside the body of the patient carrying the first intracardiac device. The first intracardiac device comprises a first processing unit for controlling a first signal generator unit, a first measurement unit for determining at least one first bodily value and a second bodily value, and a first data memory comprising pre-defined first signal parameters for a first treatment signal. The first bodily value and the second bodily value may be the same physical or chemical measurand or different physical or chemical measurand. The second implantable device comprises a second processing unit for controlling a second signal generator unit and a second data memory comprising second pre-defined signal parameters for a second treatment signal. The second device may additionally comprise a second measurement unit for determining at least one third bodily value. The first processing unit, first signal generator unit, first measurement unit and first data memory are all electrically connected and contained within the housing of the first intracardiac device. The second processing unit, second signal generator unit, second measurement unit and second data memory are all electrically connected and contained within the housing of the second device. The first processing unit and/or the second processing unit may comprise hardware to support signal processing (e.g. scaling, filtering, rectification).

According to the invention, the first signal generator unit is adapted to deliver a first electric and/or electromagnetic treatment signal to the patient's heart with at least one first signal parameter and an information carrying parameter, wherein the at least one first signal parameter of the first treatment signal is derived from the first data memory based on the at least one determined first bodily value, wherein the information carrying parameter is determined by the first processing unit based on the determined second bodily value. The first treatment signal is provided by the first signal generator and comprises a time-dependent changing electric and/or electromagnetic field applied to the cardiac tissue of the patient, for example a pacing signal, for treatment in order to improve the health condition of the patient.

The electric and/or electromagnetic field may be applied, for example, by electrodes protruding from the first intracardiac device and fixed within the tissue of the respective chamber of the heart. The electrodes are electrically connected with the first signal generator unit. The first treatment signal is characterized by its first signal parameters, for example its start time position, its amplitude and/or frequency (rate) of the signal as well as signal morphology. The first signal parameters are derived from the first data memory, wherein the chosen first signal parameters are the pre-defined signal parameters stored in the first data memory. They are chosen by the first processing unit, for example, from a data matrix containing pre-defined signal parameters with a connection to possible measurable first bodily values. According to the at least one first bodily value determined by the first measurement unit, the suitable at least one first signal parameter is chosen from the data matrix. Accordingly, the first signal parameters are chosen from the data matrix containing pre-defined signal parameters based on recently measured first bodily value (i.e. vital sign of the patient's body such as heart rate). According to the invention, the treatment signal further realizes an information carrying parameter which is determined by the first processing unit based on the determined second bodily value. The information carrying parameter is determined, for example calculated or derived from a respective data matrix of the first data memory containing pre-defined information carrying parameters, by the first processing unit based on the second bodily value determined by the first measurement unit. For example, the second bodily value may be an activity value determined by an accelerometer and/or impedance sensor of the first measurement unit thereby assessing the activity of the patient.

The second device is able to determine the information carrying value provided with the first treatment signal and relates some information to the determined value of the information carrying parameter. For example, the second device compares pre-defined treatment signals and/or pre-defined first parameters of treatment signals contained in the first data memory with the most recently (i.e. just before) measured treatment signal or determined first parameters of such treatment signal of the first intracardiac device. According to the invention, the second signal generator is adapted to deliver a second electric and/or electromagnetic treatment signal, for example to the patient's heart, wherein at least one second signal parameter of the second treatment signal is derived from second data memory based on the at least one determined third bodily value and is further based on the determined information carrying parameter of the first electric and/or electromagnetic treatment signal recently provided by the first intracardiac device. The second electric and/or electromagnetic treatment signal may comprise, for example, a time-dependent changing electric and/or electromagnetic field applied to the cardiac tissue of the patient, e.g. a pacing signal, for treatment in order to improve the health condition of the patient. The electric and/or electromagnetic field may be applied, for example, by electrodes protruding from the second device being an intracardiac device and fixed within the tissue of the respective chamber of the heart. The electrodes are electrically connected with the second signal generator unit. The second treatment signal is characterized by its second signal parameters, for example its start time position, its amplitude and/or frequency (rate) of the signal as well as signal morphology. The second signal parameters are derived from the second data memory, wherein the chosen second signal parameters are the pre-defined signal parameters stored in the second data memory. They are chosen by the second processing unit, for example, from a data matrix containing pre-defined signal parameters associated with possible measurable third bodily values. According to the at least one third bodily value determined by the second measurement unit, the suitable at least one second signal parameter is chosen from the data matrix. Accordingly, the second signal parameters are chosen from the data matrix containing pre-defined signal parameters for the second treatment signal based on the recently measured third bodily value (i.e. vital sign of the patient's body such as heart rate). The at least one second signal parameter is further adapted according to the determined information carrying parameter and prior to application of the second treatment signal to the patient. For example, the second processing unit uses the at least one signal parameter derived from the second data memory and increases, decreases or maintains at least one parameter of the at least one second signal parameters according to the most recently (i.e. from the just before provided first treatment signal) provided first treatment signal and recently determined information carrying parameter from this signal.

In one embodiment, the first and the second measurement unit are adapted to measure the patient's intracardiac electrocardiogram (IEGM) signal comprising, for example, the heart rate, PR-interval, QT-interval, ST-interval, P-wave and T-wave durations. With the IEGM signal, the first and the second measurement unit additionally may sense a pacing signal (e.g. its start time position and its duration as well as its frequency) from the respective other intracardiac device. Additionally, the first and the second measurement unit may be adapted to determine the impedance in order to derive cardiac tissue properties. The first and the second measurement unit may also sense multiple signals indicative of multiple contractions of the ventricle and determine the ventricular contraction rate from the multiple signals. Sensed signals may include, for example, far-field R-waves and/or heart sounds. Communication between at least two devices in this way may be possible by simply passively sensing the resulting electrical activity from the different devices in the heart. For example, dual chamber leadless pacer therapy, with one device sensing and pacing in the ventricle and a second sensing and pacing in the atrium, may rely on each device sensing the far-field activity from the electrically distant chamber to remain synchronized with each other. The first measurement unit further may comprise an accelerometer sensor and/or impedance sensor as indicated below.

In one embodiment the first treatment signal is an electric ventricular pacing signal, wherein the at least one first signal parameter of the first intracardiac device is the start time position of the ventricular pacing signal and/or its amplitude and/or the second treatment signal is an electric atrial pacing signal, wherein the signal parameter of the second device is an atrial pacing rate, an amplitude of the pacing signal, a duration of the pacing signal and/or the start time position of the atrial pacing signal.

In one embodiment the information carrying parameter is a time period by which a pre-defined first electric and/or electromagnetic treatment signal section is shortened or prolonged, e.g. an AV delay value is shortened or prolonged, and/or a rate of a pre-defined first electric and/or electromagnetic treatment signal. Also, the VA-interval may be modulated. This shortening or prolonging time period may be between -50 ms and 50 ms, preferably between -15 ms and 15 ms, and more preferably between -8 ms and 8 ms. The negative values are regarded as shortening time period and the positive values are regarded as prolonging time period. In a further embodiment, the shortening or prolonging time period are between -8 ms and -3 ms or between 3 ms and 8 ms. In all intervals mentioned before the respective boundary values are included. The AV delay is the time period between the start time position of the atrial pacing signal and the start time position of the ventricular pacing signal. In this embodiment, the first intracardiac device, e.g. a ventricular ILP, may sense the start time position of the atrial pacing signal produced by the second device using the first measurement unit. In addition, the second device, e.g. an atrial ILP, may sense the start time and position of the ventricular pacing signal produced by the first intracardiac device using the second measurement unit. The first and the second devices are individually programmed and contain the expected AV delays and/or pacing rate for a particular heart rate stored in the first data memory and the second data memory. In case both devices 'know' the present heart rate of the patient they may have an 'expectation' of when the ventricular pace should happen, i.e. they know the 'usual' AV delay and/or pacing rate. A shortening or prolongation of the 'usual' AV delay by the ventricular ILP and/or change in the pacing rate may be used to communicate information to the atrial device. E.g. if the ventricular device is pacing at a shorter AV delay than expected, i.e. the pacing signal of the ventricular ILP begins by a certain time period earlier or later, the atrial ILP detects the shortening or prolongation of the AV delay by comparing the measured AV delay with the pre-defined AV delay stored in its data memory. If a shortening of the AV delay is determined, the second processing unit derives the information that the atrial pacing rate shall be increased. In contrast, if the atrial pacing rate is higher than indicated by the accelerometer and/or impedance of the ventricular ILP, the ventricular ILP would delay delivering the ventricular pace, which would be an indicator for the atrial ILP to lower the pacing rate. Additionally or alternatively, in one embodiment, if a change in the ventricular pacing rate is detected, the atrial pacing is adapted accordingly so that pacing remains in synchrony with each other. Hence, in case the patient develops sequences of atrial tachycardias the two leadless pacemakers would work in an asynchrony, the accelerometer and/or impedance based sensor indicated pacing rate would be available in the ventricular ILP allowing for standard VVIR pacing.

The inventive system uses the treatment signal of the first intracardiac device for communication of information to the second device which adapts its treatment signal according to the received information. For example, it may increase or decrease the atrial pacing rate based on the changing AV delay and or changing pacing rate in the ventricle. An active communication (for example NF communication or blue tooth communication) using a separate communication channel is not necessary for communication between the first intracardiac device and the second device. Accordingly, as the separate communication channel is not used with this communication, there is no energy consumption with regard to such communication. The energy used in connection with the first treatment signal is not significantly changed. Additionally, no special modulating unit is necessary as only a small shift of the start time position of the first treatment signal contains the information. The information carrying parameter does not alter the treatment activity of the first treatment signal as its small value, compared to typical near-field electrical signals, does not influence the effectiveness of treatment but is reliably measurable by the second measurement unit.

In one embodiment, the information carrying parameter takes into account the variability in the timing of far-field signals, e.g. far-field atrial detections in the first intracardiac device (e.g. the ventricular device). For example, it is likely that the atrial and ventricular devices detect the atrial event at slightly different times due to the signal processing and detection mechanism for far-field sensing in the ventricular device. This means that the AV delay as measured by the second device (e.g. atrial intracardiac device) and the first intracardiac device (e.g. ventricular device) may be slightly different. This difference may be considered when setting tolerances for increasing or decreasing the AV delay by the second device to communicate a pacing rate.

In one embodiment the first bodily value comprises the intrinsic atrial activity and/or the intrinsic ventricular activity and/or the third bodily value comprises the intrinsic ventricular activity. For example, the first measurement unit and the second measurement unit detect the ECG or IEGM of the patient's heart, for example high resolution and high gain IEGM.

In one embodiment the first bodily value comprises the intrinsic near-field ventricular electrical activity and/or the intrinsic far-field atrial electrical activity and/or the atrial pacing delivered by the second device. The third bodily value may comprise the intrinsic near-field atrial electrical activity and/or the intrinsic far-field ventricular electrical activity and/or the ventricular pacing delivered by the first intracardiac device.

Alternatively or additionally, the first bodily value comprises the intrinsic or paced atrial activity and/or the intrinsic or paced ventricular activity and/or the third bodily value comprises the intrinsic ventricular and/or atrial activity and/or the intrinsic or paced atrial activity.

In one embodiment the second bodily value is a signal determined by an accelerometer sensor, wherein the accelerometer sensor is integrated within the first measurement unit. The accelerator sensor is, for example, adapted for measuring activity to determine the desired pacing rate, posture and/or heart sound data. Alternatively or additionally, the second bodily value is an impedance signal measured at a bodily tissue or within a bodily fluid, for example within the blood of the heart's ventricle. For example, the impedance caused by the pH value (i.e. the acidity or basicity) of the patient's blood may be a characteristic parameter with regard to the activity of the patient. The impedance sensor may be integrated within the first measurement unit.

In one embodiment the second device only allows change of the second electric and/or electromagnetic treatment signal previously applied to the patient's heart if the second measurement unit has received a or any confirmation of the most recently determined information carrying parameter. The confirmation may be provided by at least one second transmission of the information carrying parameter in a consecutive manner (i.e. an additional second time or even more than a second time, e.g. three times or four times in a consecutive manner) thereby forming a set of at least two consecutive first treatment signals, both containing the same information carrying parameter value. The number of consecutive first treatment signals comprising the same information carrying parameter values which is necessary for second treatment signal change is pre-defined for each system. This is advantageous because it enhances the safety and reliability of the system.

Further, it is of advantage that the communication of the devices is visible to the clinician or outside world. Existing surface ECG systems can be used to assess heart rhythm (such as AV delay or rate) and therefore interpret the signaling from either or both devices. This may improve troubleshooting or may be used to interpret how to program the first intracardiac device and the at least one second device, for example, how to program rate response based on observation of AV delay.

The above problem is also solved by a method of operation of a system comprising a first intracardiac device after implantation within the heart of a patient and at least one second implantable device, for example an intracardiac device,
wherein the first intracardiac device comprises a first processing unit for controlling a first signal generator unit, a first data memory comprising pre-defined signal parameters for a first treatment signal and a first measurement unit for determining at least one first bodily value and at least one second bodily value,
the second intracardiac device comprises a second processing unit for controlling a second signal generator unit and a second data memory comprising pre-defined signal parameters for a second treatment signal and a second measurement unit for determining at least one third bodily value,
wherein the method comprises the following steps:
   determining at least one first bodily value by the first measurement unit of the first intracardiac device,
   determining at least one second bodily value by the first measurement unit of the first intracardiac device,
   determining an information carrying parameter by the first processing unit based on the determined at least one second bodily value,
   delivering a first electric and/or electromagnetic treatment signal to the patient's heart with at least one first signal parameter and the information carrying parameter by the first signal generator unit, wherein the at least one first signal parameter of the first treatment signal is derived from the first data memory based on the at least one determined first bodily value,
   determining by the second intracardiac device the information carrying parameter of the first electric and/or electromagnetic treatment signal provided by the first intracardiac device, determining at least one third bodily value by the measurement unit of the second intracardiac device,
   delivering a second electric and/or electromagnetic treatment signal to the patient's heart by the second signal generator, wherein at least one second signal parameter of the second treatment signal is derived from second data memory based on the at least one determined third bodily value and is further based on the determined information carrying parameter of the first electric and/or electromagnetic treatment signal recently provided by the first intracardiac device.

The above method has the advantages as explained with regard to the system above. The embodiments mentioned and explained above with regard to the systems also apply to the method indicated above.

The present invention will now be described in further detail with reference to the accompanying schematic drawing, wherein
- Fig. 1: shows a first embodiment of the inventive system within a cross section of a patient's heart,
- Fig. 2: depicts a functional block diagram of a first intracardiac device of the embodiment shown in Fig. 1,
- Fig. 3: depicts an example of the AV delay modulation of the ventricular device to relay pace rate information to the atrial device, and
- Fig. 4: shows atrial signals over time, namely in the first row an ECG signal, in the second row an RA signal and in the third row a near field RV signal.

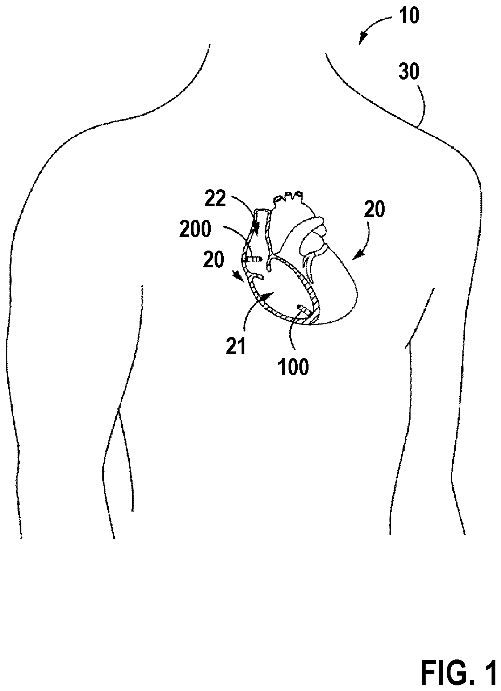

Fig. 1 shows an example leadless pacing system 10 implanted within the heart 20 of a patient 30. Leadless pacing system 10 includes a leadless ventricular pacemaker device 100 (hereinafter "ventricular device 100") and a leadless atrial pacemaker device 200 (hereinafter "atrial device 200"). Ventricular device 100 may be configured to be implanted within the right ventricle 21 of the heart and pace this ventricle, sense intrinsic ventricular depolarizations, and inhibit ventricular pacing in response to detected ventricular depolarization. Atrial device 200 may be implanted within the right atrium 22 of the heart 20 and configured to monitor the electrical activity of the heart 20 and/or provide electrical treatment to the heart 20. A programmer may be used to program ventricular device 100 and/or atrial device 200 and retrieve data from ventricular device 100 and/or atrial device 200.

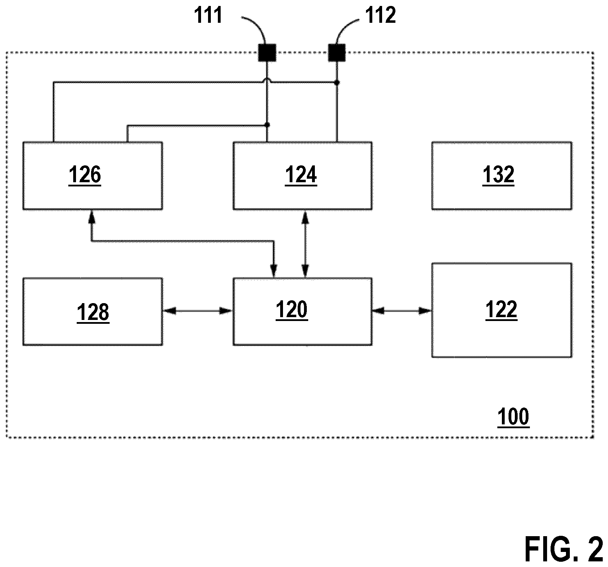

Fig. 2 shows a functional block diagram of an example ventricular device 100 configured for implantation within ventricle 21 (Fig. 1). Ventricular device 100 includes a first processing unit 120, a first data memory 122, a first signal generator unit 124, a first measurement unit 126, a first communication unit 128, and a first power source 132. The first power source 132 may include a battery, e.g., a rechargeable or non-rechargeable battery. Units included in ventricular device 100 represent functionality that may be included in ventricular device 100 of the present disclosure. Similar or identical units and functionality may also be included in a ventricular pacemaker device, which may be provided as part of a dual-chamber, leadless pacemaker system for implantation and use in at least one atrium and at least one ventricle of the heart 20. Units of the present disclosure may include any discrete and/or integrated electronic circuit components that implement analog and/or digital circuits capable of producing the functions attributed to the units herein. For example, the units may include analog circuits, e.g., amplification circuits, filtering circuits, and/or other signal conditioning circuits. The units may also include digital circuits, e.g., combinational or sequential logic circuits, memory devices, etc. The first data memory 122 may include any volatile, non-volatile, magnetic, or electrical media, such as a random access memory (RAM), read-only memory (ROM), non-volatile RAM (NVRAM), electrically-erasable programmable ROM (EEPROM), flash memory, or any other memory device. Furthermore, the first memory unit 122 may include instructions that, when executed by one or more processing circuits, cause the units to perform various functions attributed to the units herein. The functions attributed to the units herein may be embodied as one or more processors, hardware, firmware, software, or any combination thereof. Depiction of different features as units is intended to highlight different functional aspects, and does not necessarily imply that such units must be realized by separate hardware or software components. Rather, functionality associated with one or more units may be performed by separate hardware or software components, or integrated within common or separate hardware or software components. First processing unit 120 may communicate with first data memory 122. First data memory 122 may include computer-readable instructions that, when executed by processing unit 120, cause processing unit 120 to perform the various functions attributed to processing unit 120 herein. For example, memory 122 may include pacing instructions and first signal parameters for a first treatment signal, such as the baseline ventricular pacing rate, the baseline ventricular pacing interval and the baseline AV delay.

The pacing instructions and first signal parameters may be updated by the programmer using the communication unit 128.

The first processing unit 120 may communicate with first signal generator unit 124 and first measurement unit 126. First signal generator unit 124 and first measurement unit 126 are electrically coupled to electrodes 111, 112. First measurement unit 126 is configured to monitor signals from electrodes 111, 112 in order to monitor electrical activity of heart 20. Further, the first measurement unit 126 may include an accelerometer and/or a pressure sensor and/or an impedance sensor. First signal generator unit 124 is configured to deliver electrical stimulation to ventricle 21 via electrodes 111,112.

First processing unit 120 may control first signal generator unit 124 to generate and deliver electrical stimulation to ventricle 21 via electrodes 111,112. Electrical stimulation may include pacing pulses. First processing unit 120 may control first signal generator unit 124 to deliver electrical stimulation therapy according to one or more ventricular therapy programs including pacing instructions and values, which may be stored in first data memory 122.

First measurement unit 126 may include circuits that acquire electrical signals from the sensor(s). Electrical signals acquired by first measurement unit 126 may include intrinsic cardiac electrical activity, such as intrinsic ventricular and/or intrinsic ventricular cardiac electrical activity. First measurement unit 126 may filter, amplify, and digitize the acquired electrical signals to generate raw digital data. First processing unit 120 may receive the digitized data generated by First measurement unit 126. In some examples, first processing unit 120 may perform various digital signal processing operations on the raw data, such as digital filtering.

Processing unit 120 may sense cardiac events based on the data received from first measurement unit 126. For example, first processing unit 120 may determine ventricular events based on the data received from first measurement unit 126. In some examples, first processing unit 120 may determine ventricular activation based on the data received from first measurement unit 126. For example, first processing unit 120 may detect far-field R-waves (FFRWs) indicative of ventricular activation based on the data received from first measurement unit 126.

Ventricular device 100 may include a housing, fixation tines, and electrodes 111, 112. The housing may have a pill-shaped cylindrical form factor in some examples. Fixation tines are configured to connect (e.g., anchor) ventricular device 100 to heart 20. Fixation tines may be fabricated from a shape memory material, such as Nitinol. In some examples, fixation tines may connect ventricular device 100 to heart 20 within one of the chambers of heart 20. For example, as illustrated and described herein with respect to Fig. 1, fixation tines may be configured to anchor ventricular device 100 to heart 20 within right ventricle 21. Although ventricular device 100 includes a plurality of fixation tines that are configured to anchor ventricular device 100 to cardiac tissue in the right ventricle, it is contemplated that a leadless device according to the present disclosure may be fixed to cardiac tissue in other chambers of a patient's heart 20 using other types of fixation mechanisms.

Ventricular device 100 may include one or more electrodes 111, 112 for sensing electrical activity of heart 20 and/or delivering electrical stimulation to heart 20. Ventricular device 100 includes two electrodes 111,112, although more than two electrodes may be included on an ventricular device in other examples. Electrodes 111, 112 may be spaced apart a sufficient distance to be able to detect various electrical signals generated by the heart 20, such as P-waves generated by atria and FFRWs generated by ventricles. The housing houses electronic components of ventricular device 100. Electronic components may include any discrete and/or integrated electronic circuit components that implement analog and/or digital circuits capable of producing the functions attributed to ventricular device 100 described above.

The first communication unit 128 may enable leadless device 100 to communicate with other electronic devices, such as a programmer or other external patient monitor. In some examples, housing 108 may house an antenna for wireless communication. Housing may also include the first power source 132.

The structure of atrial device 200 may be similar to the structure of ventricular device 100 as described above. For example, atrial device 100 may have a housing, fixation tines, and electrodes that are similar to housing, fixation tines, and electrodes of ventricular device 100. Additionally, the atrial device 200 may have a second processing unit similar to the first processing unit 120, a second data memory similar to the first data memory 122, a second signal generating unit similar to the first signal generating unit 124, and a second measurement unit similar to the first measurement unit 126, but an accelerometer and/or an impedance sensor and/or any other sensor used to determine the patient's activity level for rate response is not needed in the atrial device. Further, the atrial device 200 may comprise a second communication module similar to the first communication unit 128 for communication with a programmer or an external patient monitor.

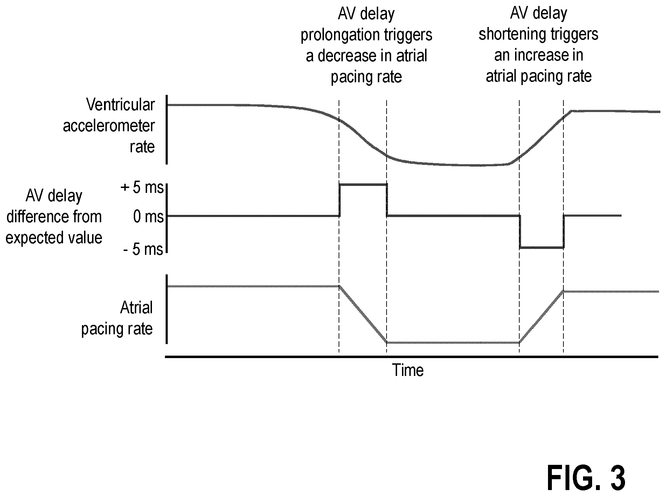

Figure 3 shows an example of how AV delay information can be used to trigger changes in the atrial pacing rate. The atrial device 200 is at least capable of sensing the intrinsic atrial activity, the ventricular activity (from far-field), and pacing the atrial cardiac tissue. The ventricular device 100 is at least capable of sensing the ventricular activity, pacing the ventricular tissue, triggering ventricular pacing based on the detection of atrial activity (from far-field), and deriving the desired atrial pacing rate based on the signal provided by the accelerometer and/or impedance sensor of the first measurement unit 126 of the ventricular device 100. Both devices 100, 200 are individually programmed, including the expected AV delays for a particular heart rate determined by the first measurement unit 126. Both devices, 100 and 200, 'know' the present heart rate of the patient 30 and have an 'expectation' of when the ventricular pace should happen because these signal parameters are stored in the first and the second data memory. Therefore, an information carrying parameter in form of a prolongation or shortening of the pre-defined (expected) AV delay by the ventricular device 100 is used to communicate simple information to the atrial device when the first treatment signal is applied by the first signal generator unit 124. E.g. if the ventricular device 100 is pacing at a shorter AV delay than expected this is used as the information that the atrial pacing rate of the atrial device 200 has to be increased. In contrast, if the atrial pacing is higher than indicated by the accelerometer and/or impedance sensor, the ventricular device 100 would delay delivering the ventricular pace, i.e. prolong the AV delay, which would be an indicator for the atrial device 200 to lower its pacing rate. The example in Figure 3 depicts a scenario where the accelerometer and/or impedance sensor in the ventricular device first indicates a need to decrease the atrial pacing rate to match demand. The decrease in the accelerometer- and/or impedance-determined rate initiates a 5 ms step increase in the AV delay difference from the expected value, that then triggers the atrial pacing rate to decrease at a constant rate until the AV delay returns to the expected value when the atrial pacing rate matches the accelerometer- and/or impedance-determined rate. After a period of a constant atrial pacing rate, the accelerometer-and/or impedance-determined rate increases, which initiates a period of a AV delay shortened by 5 ms compared to the expected value that triggers an increase in the atrial pacing rate. The atrial pacing rate stabilizes once the AV delay returns the expected value. In case the patient develops sequences of atrial tachycardias the two devices 100, 200 would be work in an asynchrony, the accelerometer and/or impedance based first measurement unit 126 indicated pacing rate would be available in the ventricular device 100 allowing for standard VVIR pacing.

In addition, based on typical programming of shorter AV delays at higher pacing rates, the proposed solution is intrinsically safe. In order to transmit the information to further increase of the desired atrial pacing rate to the atrial device 200 the ventricular device 100 would need to further shorten the paced AV delay. It is conceivable that the information carrying parameter (i.e. the value by which the AV delay is shortened or prolonged) used to communicate a change of the atrial pacing rate has to fall within predetermined values (e.g. a change of the expected AV delay by a time period between 3 ms to 8 ms (prolongation) and between - 8 ms to -3 ms (shortening) are considered a signal. Changes in AV delay which do not fall within these expected ranges would be considered noise). In one embodiment the signal transmission using the information carrying parameter has to be repeated in a specific sequence to validate the input for the atrial device 200, for example, three times consecutively.

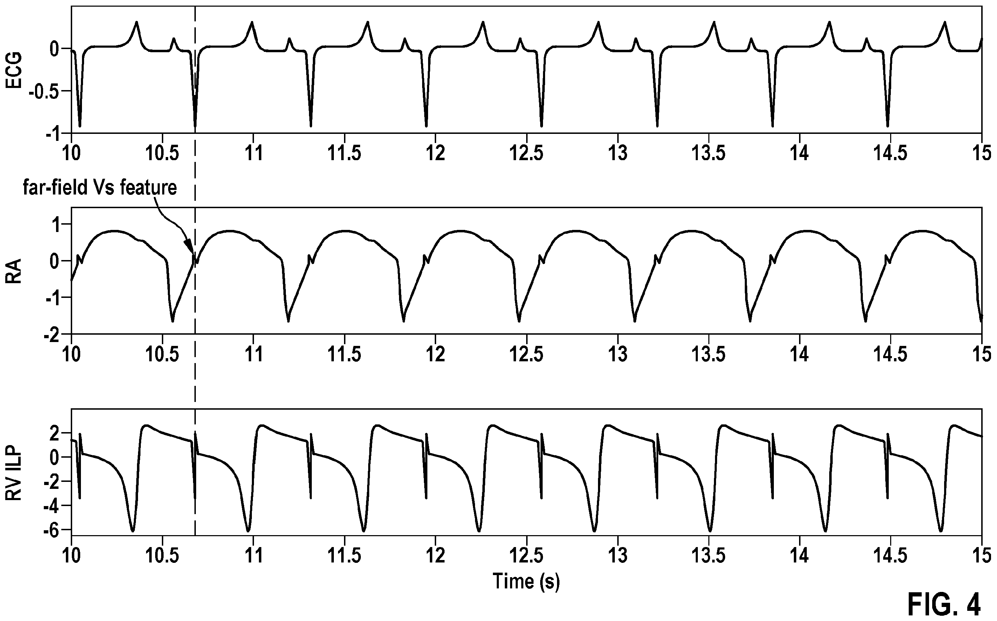

In another embodiment, communication between multiple leadless (intracardiac) pacing devices in this way may be possible by simply passively sensing the resulting electrical activity from the respective other device(s) in the heart. Dual chamber leadless pacer therapy, with the ventricular device 100 sensing and pacing in the ventricle and the atrial device 200 sensing and pacing in the atrium, relies on each device sensing the far-field activity from the electrically distant chamber to remain synchronized with each other. Far-field atrial activity is sensed by the ventricular device for VDD therapy, and preliminary preclinical data indicate that far-field electrical signals from ventricular activity are also present in the atrium. An example ventricular far-field signal recorded in the atrium is shown in Figure 4 (see second row and arrow). In this embodiment, the information carrying parameter may be, for example the pacing time and/or pacing rate.

The technical advantage of this invention is the ability to provide atrial rate responsive pacing in a leadless pacemaker system consisting of two independent devices 100, 200. The atrial device 200 can be designed simpler (less electronic components), smaller, and having a longer device lifetime, without compromising patient therapy. Further, duplication of device functionality like rate response based on an accelerometer and/or impedance sensor is avoided, for example, in the atrial device 200. Instead, the ventricular device 100 calculates the ventricular pacing rate and communicates this information to the atrial device 200.

## Claims

1. A system (10) comprising a first intracardiac device (100) for implantation within the heart (20) of a patient (30) and at least one second implantable device (200),
wherein the first intracardiac device (100) comprises a first processing unit (120) for controlling a first signal generator unit (124), a first data memory (122) comprising pre-defined first signal parameters for a first treatment signal and a first measurement unit (126) for determining at least one first bodily value and at least one second bodily value, and
the second device (200), for example an intracardiac device, comprises a second processing unit for controlling a second signal generator unit and a second data memory comprising pre-defined second signal parameters for a second treatment signal and a second measurement unit for determining at least one third bodily value, wherein the first signal generator unit (124) is adapted to deliver a first electric and/or electromagnetic treatment signal to the patient's heart (20) with at least one first signal parameter and an information carrying parameter, wherein the at least one first signal parameter of the first treatment signal is derived from the first data memory (122) based on the at least one determined first bodily value, wherein the information carrying parameter is determined by the first processing unit (120) based on the determined second bodily value,
wherein the second signal generator is adapted to deliver a second electric and/or electromagnetic treatment signal, for example to the patient's heart (20), wherein at least one second signal parameter of the second treatment signal is derived from second data memory based on the at least one determined third bodily value and is further based on the determined information carrying parameter of the first electric and/or electromagnetic treatment signal recently provided by the first intracardiac device (100).

2. The system of claim 1, wherein the first treatment signal is an electric ventricular pacing signal, wherein the at least one first signal parameter of the first intracardiac device (100) is a start time position of the ventricular pacing signal and/or its amplitude and/or a ventricular pacing rate and/or wherein the second treatment signal is an electric atrial pacing signal, wherein the second signal parameter of the second device (200) is an atrial pacing rate, an amplitude of the pacing signal and a start time position of the atrial pacing signal.

3. The system of any of claims 1 to 2, wherein the information carrying parameter is a time period by which a pre-defined first electric and/or electromagnetic treatment signal section is shortened or prolonged, e.g. an AV delay value is shortened or prolonged, and/or a rate of a pre-defined first electric and/or electromagnetic treatment signal.

4. The system of any of the previous claims, wherein the first bodily value comprises the intrinsic atrial activity and/or the intrinsic ventricular activity and/or the third bodily value comprises the intrinsic ventricular activity and/or the intrinsic atrial activity.

5. The system of any of the previous claims, wherein the second bodily value is a signal determined by an accelerometer sensor and/or impedance sensor of the first measurement unit (126).

6. The system of any of the previous claims, wherein the second device (200) only allows change of the second electric and/or electromagnetic treatment signal previously applied to the patient's heart (20) if the second measurement unit has received confirmation of the recently determined information carrying parameter.

## Patentansprüche

1. System (10), umfassend eine erste intrakardiale Vorrichtung (100) zur Implantation innerhalb des Herzens (20) eines Patienten (30) und mindestens eine zweite implantierbare Vorrichtung (200), wobei die erste intrakardiale Vorrichtung (100) eine erste Verarbeitungseinheit (120) zum Steuern einer ersten Signalgeneratoreinheit (124), einen ersten Datenspeicher (122), der vordefinierte erste Signalparameter für ein erstes Behandlungssignal umfasst, und eine erste Messeinheit (126) zum Bestimmen von mindestens einem ersten Körperwert und mindestens einem zweiten Körperwert, umfasst und
die zweite Vorrichtung (200), zum Beispiel eine intrakardiale Vorrichtung, eine zweite Verarbeitungseinheit zum Steuern einer zweiten Signalgeneratoreinheit und einen zweiten Datenspeicher, der vordefinierte zweite Signalparameter für ein zweites Behandlungssignal umfasst, und eine zweite Messeinheit zum Bestimmen von mindestens einem dritten Körperwert umfasst, wobei die erste Signalgeneratoreinheit (124) dazu ausgelegt ist, ein erstes elektrisches und/oder elektromagnetisches Behandlungssignal an das Herz (20) des Patienten mit mindestens einem ersten Signalparameter und einem informationstragenden Parameter abzugeben, wobei der mindestens eine erste Signalparameter des ersten Behandlungssignals von dem ersten Datenspeicher (122) basierend auf dem mindestens einen bestimmten ersten Körperwert abgeleitet wird, wobei der informationstragende Parameter von der ersten Verarbeitungseinheit (120) basierend auf dem bestimmten zweiten Körperwert bestimmt wird, wobei der zweite Signalgenerator dazu ausgelegt ist, ein zweites elektrisches und/oder elektromagnetisches Behandlungssignal abzugeben, zum Beispiel an das Herz (20) des Patienten, wobei mindestens ein zweiter Signalparameter des zweiten Behandlungssignals von dem zweiten Datenspeicher basierend auf dem mindestens einen bestimmten dritten Körperwert abgeleitet wird und ferner auf dem bestimmten informationstragenden Parameter des ersten elektrischen und/oder elektromagnetischen Behandlungssignals basiert, das kürzlich von der ersten intrakardialen Vorrichtung (100) bereitgestellt wurden.

2. System nach Anspruch 1, wobei das erste Behandlungssignal ein elektrisches ventrikuläres Stimulationssignal ist, wobei der mindestens eine erste Signalparameter der ersten intrakardialen Vorrichtung (100) eine Startzeitposition des ventrikulären Stimulationssignals und/oder dessen Amplitude und/oder eine ventrikuläre Stimulationsrate ist und/oder wobei das zweite Behandlungssignal ein elektrisches atriales Stimulationssignal ist, wobei der zweite Signalparameter der zweiten Vorrichtung (200) eine atriale Stimulationsrate, eine Amplitude des Stimulationssignals und eine Startzeitposition des atrialen Stimulationssignals ist.

3. System nach einem der Ansprüche 1 bis 2, wobei der informationstragende Parameter ein Zeitraum ist, in dem ein vordefinierter erster elektrischer und/oder elektromagnetischer Behandlungssignalabschnitt verkürzt oder verlängert wird, z. B. ein AV-Verzögerungswert verkürzt oder verlängert wird, und/oder eine Rate eines vordefinierten ersten elektrischen und/oder elektromagnetischen Behandlungssignals ist.

4. System nach einem der vorhergehenden Ansprüche, wobei der erste Körperwert die intrinsische atriale Aktivität und/oder die intrinsische ventrikuläre Aktivität umfasst und/oder der dritte Körperwert die intrinsische ventrikuläre Aktivität und/oder die intrinsische atriale Aktivität umfasst.

5. System nach einem der vorhergehenden Ansprüche, wobei der zweite Körperwert ein Signal ist, das von einem Beschleunigungsmessersensor und/oder Impedanzsensor der ersten Messeinheit (126) bestimmt wird.

6. System nach einem der vorhergehenden Ansprüche, wobei die zweite Vorrichtung (200) nur eine Änderung des zweiten elektrischen und/oder elektromagnetischen Behandlungssignals ermöglicht, das vorher an das Herz (20) des Patienten angelegt wurde, wenn die zweite Messeinheit eine Bestätigung des kürzlich bestimmten informationstragenden Parameters empfangen hat.

## Revendications

1. Système (10) comprenant un premier dispositif intracardiaque (100) pour implantation à l'intérieur du cœur (20) d'un patient (30) et au moins un second dispositif implantable (200), dans lequel le premier dispositif intracardiaque (100) comprend une première unité de traitement (120) destinée à commander une première unité de générateur de signal (124), une première mémoire de données (122) comprenant des paramètres de premier signal prédéfinis pour un premier signal de traitement et une première unité de mesure (126) destinée à déterminer au moins une première valeur corporelle et au moins une deuxième valeur corporelle, et
le second dispositif (200), par exemple un dispositif intracardiaque, comprend une seconde unité de traitement destinée à commander une seconde unité de générateur de signal et une seconde mémoire de données comprenant des paramètres de second signal prédéfinis pour un second signal de traitement et une seconde unité de mesure destinée à déterminer au moins une troisième valeur corporelle, dans lequel la première unité de générateur de signal (124) est adaptée pour distribuer un premier signal de traitement électrique et/ou électromagnétique au cœur du patient (20) avec au moins un paramètre de premier signal et un paramètre de transport d'information, dans lequel l'au moins un paramètre de premier signal du premier signal de traitement est dérivé de la première mémoire de données (122) en se basant sur l'au moins une première valeur corporelle déterminée, dans lequel le paramètre de transport d'information est déterminé par la première unité de traitement (120) en se basant sur la deuxième valeur corporelle déterminée,
dans lequel le second générateur de signal est adapté pour distribuer un second signal de traitement électrique et/ou électromagnétique, par exemple au cœur du patient (20), dans lequel au moins un paramètre de second signal du second signal de traitement est dérivé d'une seconde mémoire de données en se basant sur l'au moins une troisième valeur corporelle déterminée et en se basant en outre sur le paramètre de transport d'information déterminé du premier signal de traitement électrique et/ou électromagnétique récemment fourni par le premier dispositif intracardiaque (100).

2. Système selon la revendication 1, dans lequel le premier signal de traitement est un signal de stimulation ventriculaire électrique, dans lequel l'au moins un paramètre de premier signal du premier dispositif intracardiaque (100) est une position de temps de départ du signal de stimulation ventriculaire et/ou son amplitude et/ou une fréquence de stimulation ventriculaire et/ou dans lequel le second signal de traitement est un signal de stimulation auriculaire électrique, dans lequel le paramètre de second signal du second dispositif (200) est une fréquence de stimulation auriculaire, une amplitude du signal de stimulation et une position de temps de départ du signal de stimulation auriculaire.

3. Système selon l'une quelconque des revendications 1 à 2, dans lequel le paramètre de transport d'information est une période de laquelle une section de premier signal de traitement électrique et/ou électromagnétique prédéfini est raccourcie ou prolongée, p. ex. une valeur de retard AV est raccourcie ou prolongée, et/ou une fréquence d'un premier signal de traitement électrique et/ou électromagnétique prédéfini.

4. Système selon l'une quelconque des revendications précédentes, dans lequel la première valeur corporelle comprend l'activité auriculaire intrinsèque et/ou l'activité ventriculaire intrinsèque et/ou la troisième valeur corporelle comprend l'activité ventriculaire intrinsèque et/ou l'activité auriculaire intrinsèque.

5. Système selon l'une quelconque des revendications précédentes, dans lequel la deuxième valeur corporelle est un signal déterminé par un capteur à accéléromètre et/ou un capteur d'impédance de la première unité de mesure (126).

6. Système selon l'une quelconque des revendications précédentes, dans lequel le second dispositif (200) permet uniquement le changement du second signal de traitement électrique et/ou électromagnétique précédemment appliqué au cœur du patient (20) si la seconde unité de mesure a reçu une confirmation du paramètre de transport d'information récemment déterminé.
